# EUROPÄISCHE PATENTSCHRIFT

(11) **EP 1 103 538 B1**
(45) Veröffentlichungstag und Bekanntmachung des Hinweises auf die Patenterteilung: **28.05.2003**
(21) Anmeldenummer: 00121938.5
(22) Anmeldetag: 09.10.2000
(51) Int. Cl.: C07C 45/74, C07C 45/75

(54) **Verfahren zur Durchführung von Aldolkondensationen**
Process for carrying out aldolcondensations
Procédé pour la mise en oeuvre de condensations aldoliques

(30) Priorität: 24.11.1999 DE 19956410
(43) Veröffentlichungstag der Anmeldung: 30.05.2001
(73) Patentinhaber: Oxeno Olefinchemie GmbH, 45764 Marl (DE)
(72) Erfinder: Protzmann, Guido, Dr., 45772 Marl (DE); Wiese, Klaus-Diether, Dr., 45721 Haltern (DE); Büschken, Wilfried, Dr., 45721 Haltern (DE)

(56) Entgegenhaltungen:
- WO-A-93/20034
- GB-A- 909 941
- GB-A- 1 116 037
- DATABASE WPI Section Ch, Week 197814 Derwent Publications Ltd., London, GB; Class E17, AN 1978-25882A XP002161907 & JP 50 160215 A (MITSUBISHI CHEM IND LTD) , 25. Dezember 1975 (1975-12-25)

## Beschreibung

Die vorliegende Erfindung betrifft ein Verfahren zur Durchführung von Aldolkondensationen und die Verwendung der Aldolkondensationsprodukte.

Aldolkondensationen sind wichtige, großtechnisch durchgeführte Reaktionen. Die hieraus erhaltenen α,β-ungesättigten Carbonylverbindungen sind wegen ihrer Reaktivität Ausgangspunkt für die Synthese vieler organischer Stoffe, beispielsweise Zwischenprodukte für die Herstellung von Riechstoffen oder Pharmazeutika. α,β-ungesättigte Aldehyde können zudem zu den gesättigten Aldehyden hydriert werden, die u. a. zu den entsprechenden Carbonsäuren oxidiert werden können. Diese finden Einsatz zur Herstellung von Schmiermitteln, Sikkativen, Perestern oder Kunststoffstabilisatoren. Die vollständige Hydrierung der α, β-ungesättigten Aldehyde liefert gesättigte primäre Alkohole, die zur Erzeugung von Detergentien, Weichmachern oder als Solventien dienen.

Unter Aldolkondensation versteht man die Umsetzung von zwei Ketoverbindungen (Aldehyd oder Keton) unter Wasserabspaltung zu einer Verbindung, die sowohl eine olefinische Doppelbindung als auch eine Carbonylfunktion enthält. Wird beispielsweise nur ein Aldehyd als Edukt eingesetzt, entsteht ein ungesättigter Aldehyd mit der doppelten Anzahl an Kohlenstoffatomen als der Ausgangsaldehyd. Dieser Reaktionstyp wird durch Säuren und Basen katalysiert. Die technischen Prozesse bevorzugen Basen, vor allem anorganische Basen wie NaOH.

Der bei weitem technisch bedeutendste Prozess dieser Art ist die Kondensation von n-Butyraldehyd zu 2-Ethylhex-2-enal, einem Zwischenprodukt für die Herstellung von 2-Ethylhexanol, einem Weichmacheralkohol.
Eine mögliche Ausführungsweise dieser Reaktion wird im SRI-Report 21 C beschrieben: Die Reaktion wird in zwei hintereinander geschalteten Kondensationsreaktoren durchgeführt. Als Katalysator wird eine zweiprozentige Natronlauge eingesetzt. Die Verweilzeit in beiden Reaktoren beträgt jeweils ca. 14 Minuten. Die Reaktionstemperatur wird durch Kühlung im ersten Reaktor auf 85 °C und im zweiten Reaktor auf 90 °C gehalten, d. h. die Reaktion ist nicht adiabatisch und die durch die Reaktion entstandene Wärmetönung muß abgeführt werden. Das Reaktionsgemisch wird anschließend durch Phasentrennung in einem Absitzbehälter in eine wäßrige Katalysatorlösung und eine organische Phase getrennt. Die Katalysatorphase wird wieder in den ersten Reaktor zurückgeführt. Ein Teil des Katalysators wird zur Abtrennung von Nebenprodukten und Reaktionswasser ausgeschleust und durch frische Katalysatorlösung ersetzt. Die abgetrennte organische Produktphase wird mit Wasser basenfrei gewaschen. Das Waschwasser wird in den ersten Reaktor gepumpt. Aus dem Rohprodukt werden Wasser und n-Butyraldehyd detillativ abgetrennt und in den ersten Reaktor zurückgeführt. Das von diesen Leichtsiedem befreite Produkt kann als solches verwendet werden oder destillativ zum Reinprodukt (2-Ethylhex-2-enal) aufgearbeitet werden.

Nach DE 3530839 wird die Kondensation von n-Butyraldehyd zu 2-Ethylhex-2-enal unter Katalyse von 0,5-5 %iger Natronlauge bei Temperaturen von 100-170 °C unter erhöhtem Druck in einem Strömungsrohr durchgeführt. Die Verweilzeit beträgt 0,2-5 Minuten. Der Reaktionsaustrag wird nach Abkühlung auf 60 °C durch Phasentrennung in die Katalysatorphase und Produktphase getrennt, wobei die Katalysatorphase nach Teilausschleusung und Ergänzung mit frischer Katalysatorlösung wieder in das Strömungsrohr zurückgeführt wird.

Nachteilig ist, daß das Reaktionswasser durch Ausschleusung von Katalysatorlösung entfernt wird. Dieser Strom ist somit wesentlich größer als derjenige, der allein für die Abtrennung der durch Canizzaroreaktion entstandenen Carbonsäuren notwendig wäre. Damit ergibt sich ein hoher Katalysatorverbrauch. Die ausgeschleuste Natronlauge enthält eine organische Fracht, sie muß daher aufgearbeitet oder in einem Klärwerk entsorgt werden, wodurch zusätzliche Kosten entstehen.

Analog zu 2-Ethylhexenal wird Decenal, eine Vorstufe für den Weichmacheralkohol Decanol (Hauptbestandteil Isopropylheptanol), durch Aldolkondensation von C₅-Aldehyden hergestellt. Verschiedene Verfahren hierzu sind z. B. in DE 4 243 524, EP 562 450, EP562 451, EP 646 563 oder DE 4 243 524 beschrieben.

Nach EP 562 451 und EP 646 563 erfolgt die Aldolkondensation von Valeraldehyd auf konventionelle Weise, d. h. analog zur Herstellung von 2-Ethylhex-2-enal in SRI 21 C, beschrieben. Es gelten daher auch die gleichen Nachteile.

Ein weiteres, kontinuierliches Aldolkondensationsverfahren wird in EP 634 994 offenbart. Dieses Verfahren gliedert sich in folgende Schritte:
a) Der Einsatzaldehyd und die wäßrige Katalysatorlösung werden in einen nichtadiabatisch gefahrenen Rührreaktor eingespeist.
b) Das aus dem Rührreaktor erhaltene Reaktionsgemisch wird in den mittleren Teil einer Destillationskolonne geleitet.
c) Als Kopfprodukt der Destillation wird ein dampförmiges Gemisch aus Edukt und Wasser erhalten, das sich nach Kondensation in eine organische Oberphase und eine wäßrige Unterphase trennt.
d) Ein Teil der wäßrigen Phase wird ausgeschleust.
e) Die organische Oberphase wird in den Reaktor zurückgeführt.
f) Als Sumpfprodukt der Destillation fällt ein Gemisch an, das die wäßrige Katalysatorlösung, Produkt und Nebenprodukte (Höhere Aldoladditions- oder Aldolkondensationsprodukte, Carbonsäuren und Alkohole, entstanden durch Canizzarro-Reaktion) enthält.
g) Das Sumpfprodukt wird gekühlt.
h) Das abgekühlte Sumpfprodukt trennt sich in zwei Phasen. Die organische Oberphase enthält das Produkt, höhermolekulare Folgeprodukte und geringe Mengen an Katalysatorlösung. Die Unterphase ist die wäßrige Katalysatorlösung, die die als Nebenprodukt entstandene Carbonsäure als Salz enthält und mit Produkt gesättigt ist.
i) Die abgetrennte Katalysatorphase wird in den Reaktor zurückgeführt.
h) Die Produktphase (Oberphase) wird abgefahren.

Dieses Verfahren hat mehrere Nachteile:
a) Die Energiebilanz kann verbessert werden, da die Reaktionswärme nicht genutzt wird. Die Reaktionswärme muß durch Kühlen des Reaktors kontrolliert werden, andererseits erfordert die Destillation des Reaktoraustrags Energie. Zur Kühlung des Destillationssumpfprodukt wird ein Kühlmedium gebraucht.
b) Während der Destillation wird das durch die Katalysatorphase basische Reaktionsgemisch thermisch belastet, wodurch die Bildung von Nebenprodukten durch Cannizzaro-Reaktion begünstigt wird und somit die Ausbeute sinkt. Dadurch bedingt muß eine größere Menge an Katalysatorlösung ausgeschleust und durch frische ersetzt werden, um die Konzentration an Carbonsäuresalzen konstant zu halten.
c) Das rohe Endprodukt wird ungewaschen aus der Anlage gefahren. Es enthält daher noch geringe Mengen an Katalysator, was Verlust an Katalysator bedeutet. Weiterhin kann der mitgeschleppte Katalysator bei Lagerung des Rohprodukts eine Verschlechterung der Produktqualität bewirken. Bei Verwendung des Produktes in einer chemischen Synthese, z. B. Hydrierung, können diese Katalysatorreste störend sein.

Es bestand daher die Aufgabe, ein Verfahren zur Kondensation von Ketoverbindungen zu α,β-ungesättigten Ketoverbindungen zu entwickeln, das umweltfreundlicher und wirtschaftlicher als die bekannten Verfahren ist.

Gegenstand der vorliegenden Erfindung ist daher ein Verfahren zur Herstellung von α, β-ungesättigten Ketoverbindungen durch basisch katalysierte Aldolkondensation von Aldehyden und/oder Ketonen mit 1 bis 15 Kohlenstoffatomen mit einer wäßrigen Katalysatorlösung in einer adiabatischen Reaktion, Trennen der so erhaltenen Reaktionsmischung in einer Kurzdestillation in ein Kopfprodukt, umfassend Wasser, Aldehyd und/oder Keton und in ein Sumpfprodukt, umfassend α, β-ungesättigte Ketoverbindungen und wäßrige Katalysatorphase, wobei die Verweilzeit der Reaktionsmischung in der Kurzdestillationsstufe maximal eine Minute beträgt.

Das erfindungsgemäße Verfahren ist für die Umsetzung von allen Ketoverbindungen oder Gemischen von Ketoverbindungen geeignet, die Aldolkondensationsreaktionen eingehen können. Wird nur eine Ketoverbindung eingesetzt, so muß diese zwei α-Wasserstoffatome (in Nachbarschaft zur CO-Gruppe) am gleichem C-Atom besitzen. Werden zwei oder mehr unterschiedliche Ketoverbindungen verwendet, so muß mindestens eine der Verbindungen zwei α-Wasserstoffatome am gleichen C-Atom haben.

Für das erfindungsgemäße Verfahren besonders geeignet sind die folgenden Verbindungen:
Ketoverbindungen mit zwei α-Wasserstoffatomen am gleichen C-Atom: Acetaldehyd, Propanal, n-Butyraldehyd, n-Valeraldehyd, 3-Methylbutyraldehyd, n-Hexanal, 3-Methylpentanal, 4-Methylpentanal, n-Heptanal, n-Octanal, n-Nonanal, n-Decanal, Aceton, Methylethylketon, Cyclohexanon, Acetophenon.

Beispiele für Ketoverbindungen mit einem α-Wasserstoffatom am gleichen C-Atom sind: Isobutyraldehyd, 2-Methylbutyraldehyd, 2-Methylpentanal, 2-Ethylhexanal, Cyclohexylcarbaldehyd, Phenyl-2-propylketon.

Beispiele für Ketoverbindungen mit keinem α-Wasserstoffatom sind: Benzaldehyd, 2.2-Dimethylpropanal, Benzophenon.
Bevorzugt werden Ketoverbindungen mit 1 bis 15 C-Atomen und/oder deren Gemische eingesetzt. Als Aldehyde werden insbesondere diejenigen, die durch Hydroformylierung von Olefinen erzeugt worden sind, verwendet. Bevorzugte Einsatzstoffe sind: n-Butyraldehyd, n-Valeraldehyd, ein Gemisch aus n-Butyraldehyd und Isobutyraldehyd, Gemische aus n-Valeraldehyd mit 2-Methylbutyraldehyd oder 3-Methylbutyraldehyd oder das entsprechende Dreikomponentengemisch. Ebenfalls kann ein Gemisch aus C₄- und C₅-Aldehyden oder ein Gemisch der isomeren Nonanale eingesetzt werden.

Als Katalysator können Hydroxide, Hydrogencarbonate, Carbonate, Carboxylate oder ihre Gemische in Form ihrer Alkali- oder Erdalkaliverbindungen oder tertiäre Amine jeweils als wäßrige Lösungen verwendet werden. Vorzugsweise kommen als wäßrige Katalysatorlösungen Alkalilaugen wie Natronlauge zum Einsatz.

Die Konzentration des basischen Katalysators in der wäßrigen Katalysatorlösung liegt in der Regel zwischen 0,1 und 10 Gew.-%, insbesondere zwischen 0,1 und 3 Gew.-%. Da bei der Umsetzung Wasser entsteht, ist die Konzentration der Katalysatorlösung im Reaktorzulauf höher als im Reaktorablauf. Aufgrund der als Nebenreaktion ablaufenden Cannizzaro-Reaktion entstehen aus dem Edukt und in geringerem Maße aus dem Produkt Alkohole und Carbonsäuren, die sich in der Katalysatorphase in Form ihrer Salze anreichern. Durch Ausschleusung eines Teils der Katalysatorlösung und durch Ersetzen mit einer äquivalenten Menge an Frischlauge kann die Konzentration der Carbonsäuresalze in der wäßrigen Katalysatorlösung zwischen 5 und 40 Gew.-% gehalten werden.

Der Anteil der wäßrigen Katalysatorlösung bezogen auf die organische Eduktphase kann in weiten Grenzen schwanken. Wird im erfindungsgemäßen Verfahren ein Rohrreaktor verwendet, so bieten sich Massenverhältnisse von organischer zu Katalysatorphase von mindestens 1: 2, bevorzugt größer als 1 : 10 an. Analoges gilt für den Einsatz von Rührkesseln.

In den speziellen Ausführungsformen der vorliegenden Erfindung wird die Konzentration der Katalysatorlösung durch Ausschleusungs- oder Rückführungsmaßnahmen kontrolliert.

Die Temperatur der Reaktionsmischung am Reaktorausgang liegt zweckmäßig über dem Siedepunkt der wäßrigen Katalysatorlösung zwischen 80 °C und 180 °C. Bei Verwendung eines Rührkessels entspricht dies der Temperatur des Reaktionsgemisches. Im Strömungsrohr bzw. Rohrreaktor wird aufgrund der adiabatischen Reaktionsführung diese Temperatur erst am Reaktorende erreicht. Unabhängig von der Art des Reaktors wird das erfindungsgemäße Verfahren adiabatisch durchgeführt.

Der Druck in der Reaktionsvorrichtung ist bedingt durch die Dampfdrücke der Komponenten im Reaktionsgemisch bei den entsprechenden Temperaturen. Die erfindungsgemäße Aldolkondensation wird bevorzugt zwischen 1,1 und 20 bar ausgeübt.

Die Reaktionsvorrichtung der erfindungsgemäßen Aldolkondensation kann mindestens ein Rührkessel bzw. eine Rührkesselkaskade oder mindestens ein Rohrreaktor bzw. Strömungsrohr sein. In jedem Reaktortyp kann mit Hilfe von Rühreinrichtungen oder statischen Mischern für eine intensive Vermischung der beiden Phasen gesorgt werden.

Das oder die Edukt(e) werden gemeinsam oder getrennt mit der wäßrigen Katalysatorlösung, optional mit der organischen Phase des Kopfproduktes der Kurzdestillation, in den adiabatisch geführten Reaktor eingespeist.

Das den Reaktor verlassene Reaktionsgemisch wird in eine Kurzdestillationsapparatur entspannt, vorzugsweise auf Normaldruck. Bei hochsiedenden Edukten kann in ein leichtes Vakuum (0,1-1bar) entspannt werden.

Die Kurzdestillation kann als Flashdestillation, als Destillation in einem Fallfilmverdampfer, als Destillation in einem Dünnschichtverdampfer oder als Destillation in einem kombinierten Fallfilm/Dünnschichtverdampfer durchgeführt werden. Die im folgenden beschriebene Flashdestillation stellt die bevorzugte, weil technisch einfachste Variante dar. Die Kurzdestillation soll das Reaktionsprodukt einer möglichst geringen thermischen und chemischen Belastung durch den Katalysator aussetzen und wird daher mit Verweilzeiten von maximal einer Minute durchgeführt. Vergleichbare Destillationen weisen Verweilzeiten von über 5 Minuten auf. Die Kurzdestillation, inbesondere die Flashung wird bevorzugt adiabatisch vorgenommen, dadurch ist die Temperatur des Sumpfproduktes niedriger als die des Zulaufs.

Das Reaktionsgemisch wird durch die Kurzdestillation in ein Kopfprodukt, umfassend Wasser, Aldehyd und/oder Keton (Edukt) und ein Sumpfprodukt, **umfassend α, β-ungesättigte Ketoverbindungen und wäßrige** Katalysatorphase getrennt.

Das Kopfprodukt erhält neben dem bereits genannten Gemisch aus Wasser und Edukt gegebenenfalls sonstige Leichtsieder (z.B. der dem Edukt entsprechende **Alkohol) und geringe Mengen an α, β-ungesättigten Ketoverbindungen. Das** Sumpfprodukt enthält neben dem Gemisch aus α, β-ungesättigten Ketoverbindungen und Katalysatorphase gegebenenfalls höhere Kondensationsprodukte, Produkte aus der Cannizarro-Reaktion der Edukte und geringe Mengen an Edukten.

Das bevorzugt ungekühlte Sumpfprodukt aus der Kurzdestillation kann in einem Absitzbehälter in eine organische Phase (Produktphase) und eine wäßrige Phase, d. h. die wäßrige Katalysatorphase getrennt werden.

Die organische Produktphase wird nach Auswaschung von Katalysatorspuren mit Wasser, vorzugsweise unter Verwendung der wäßrigen Phase des Kopfproduktes **der Kurzdestillation, aus dem Verfahren entfernt. Dieses Rohprodukt kann direkt** für weitere Umsetzungen, z.B. Hydrierung, eingesetzt werden. Optional können zusätzlich Hochsieder (höhere Aldoladdition- und Aldolkondensationsprodukte) abgetrennt und zumindestens teilweise in den Kondensationsreaktor zurückgefahren werden.

Die wäßrige Katalysatorphase wird, gegebenenfalls zusammen mit anfallendem Waschwasser, in die Aldolkondensationsreaktion zurückgefahren. Aus der Katalysatorphase kann zur Konstanthaltung des Nebenproduktspiegels ein geringer Teil ausgeschleust und durch eine äquivalente Menge an Frischkatalysator ersetzt werden.

Das Kopfprodukt der Kurzdestillation wird bei einer Temperatur, die sowohl unter dem Siedepunkt des Wassers als auch unter dem eines Minimumazeotrops liegt, kondensiert. Es entsteht ein Flüssigkeitsgemisch, das in eine organische Phase und eine wäßrige Phase getrennt werden kann.

Die organische Phase des Kopfproduktes wird optional in den Aldolkondensations-Reaktor zurückgepumpt, gegebenenfalls wird ein Teil ausgeschleust.

Ein Teil der wäßrigen Unterphase kann z. B. für die Wäsche der Produktphase, wie bereits oben erwähnt worden ist, verwendet werden.

Der andere Teil der wäßrigen Phase des Kopfprodukts oder die gesamte wäßrige Phase dient zur Ausschleusung des Reaktionswassers. In der wäßrigen Phase sind noch organische Stoffe, vor allem Edukt, gelöst. Das Abwasser kann direkt oder nach Vorreinigung zum Klärwerk gegeben werden. Die Vorreinigung kann durch Dampfstrippung oder durch azeotrope Abdestillation von organischen Stoffen erfolgen.

Die durch das erfindungsgemäße Verfahren hergestelhen Aldolkondensationsprodukte können nach Hydrierung zu den ungesättigten Alkoholen insbesondere als Detergenzien oder Weichmacheralkohol verwendet werden.

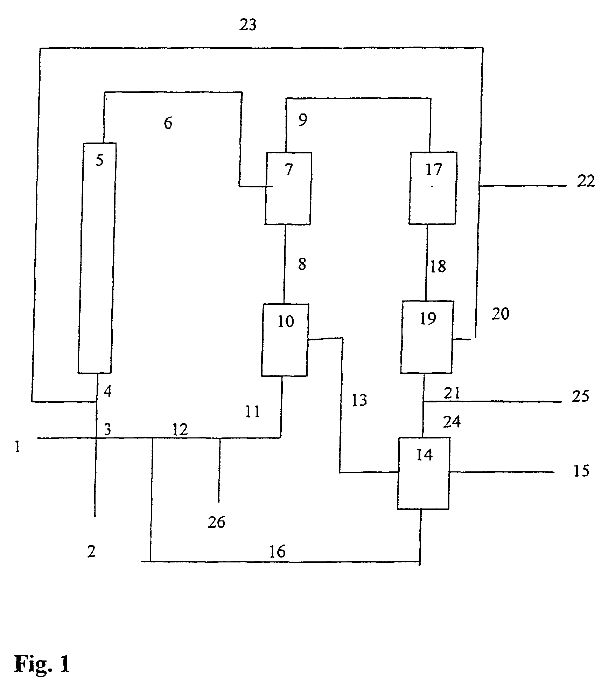

Das erfindungsgemäße Verfahren der Aldolkondensation wird bevorzugt kontinuierlich betrieben. In Figur 1 ist exemplarisch ein Blockschema einer Anlage dargestellt, in der das erfindungsgemäße Verfahren durchgeführt werden kann.

In den Reaktor 5 wird ein Gemisch 4 eingespeist, das aus Edukt 1, gegebenenfalls zurückgeführter organischer Phase 23, wäßrige Katalysatorlösung 2, zurückgefuhrter Katalysatorlösung 12 und Waschwasser 16 besteht. Das den Reaktor verlassende Reaktionsgemisch 6 wird in der Kurzdestillationsapparatur, hier ein Flashbehälter 7 entspannt. Dabei fällt ein Kopfprodukt 9 und ein Sumpfprodukt 8 an. Das Sumpfprodukt 8 wird im Absitzbehälter 10 in die Produktphase 13 und die Katalysatorphase 11 getrennt, die gegebenenfalls nach Ausschleusung einer Teilmenge 26 in den Reaktor 5 zurückgeführt wird. Die Produktphase 13 wird im Wäscher 14 mit Wasser 24 aus dem Absitzbehälter 19 gewaschen. Die Produktphase 15 verläßt die Anlage. Das Waschwasser 16 wird in den Reaktor 5 zurückgeführt. Das gasförmige Kopfprodukt 9 wird im Kühler 17 kondensiert. Das Kondensat 18 wird im Absitzbehälter 19 in eine organische Phase 20 und eine wäßrige Phase 21 getrennt. Die organische Phase 20 wird nach Ausschleusung einer Teilmenge 22 als Strom 23 in den Reaktor 5 geleitet. Aus der wäßrigen Phase 21 wird das Reaktionswasser 25 entfernt und der Rest 24 zum Waschen der Produktphase im Behälter 14 verwendet.

Das erfindungsgemäße Verfahren hat gegenüber Literatur bekannten Verfahren bemerkenswerte Vorteile: Durch die adiabatische Reaktionsführung bleibt die Reaktionswärme im Reaktionsgemisch. Diese wird in der Kurzdestillation zur Verdampfung des Kopfproduktes, d. h. von Wasser und nicht umgesetztem Edukt genutzt. Die Wärmeverluste des Verfahrens werden weiterhin dadurch minimiert, daß der Strom 8 im Behälter 10 ungekühlt getrennt und die Katalysatorphase 11 heiß in den Reaktor zurückgefahren werden kann.

Gegenüber dem in EP 0 634 994 B beschriebenen Verfahren bestehen zusätzlich folgende Vorteile: Das Reaktionsgemisch wird, da es sich nur kurze Zeit in den Behältern 7 und 10 aufhält, wesentlich kürzer in Gegenwart der alkalischen Katalysatorlösung thermisch und chemisch belastet als bei einer fraktionierten Destillation. Dadurch entsteht ein geringerer Anteil an Carbonsäure durch Cannizzaro-Reaktion. Dies bedingt eine höhere Ausbeute an Produkt. Da weniger Carbonsäure, die in der Katalysatorlösung als Salz vorliegt, ausgeschleust werden muß, ist der Katalysatorverlust geringer. Weiterhin ist es vorteilhaft, ein Teil des abdestillierten Wassers zur Wäsche des Rohprodukts zu nutzen.

Im Vergleich zu den konventiellen Verfahren, bei denen das Reaktionswasser in der Katalysatorlösung verbleibt und mit ihr ausgeschleust wird, hat das erfindungsgemäße Verfahren den zusätzlichen Vorteil eines geringeren Katalysatorverbrauchs.

Die folgenden Beispiele sollen die Erfindung näher erläutern, ohne deren Schutzumfang zu beschränken, der aus den Patentansprüchen hervorgeht.

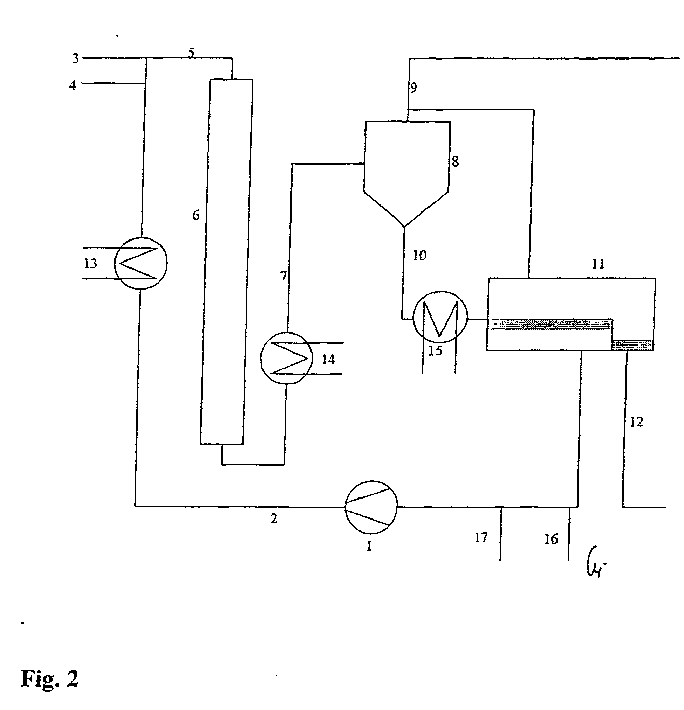

Die Aldolkondensation erfolgt in einer Versuchsapparatur, die schematisch in Fig. 2 dargestellt ist. Hierin wird mit einer Pumpe 1 die Katalysatorphase 2 im Kreislauf gepumpt. Zum Katalysator wird ein Aldehyd oder die Aldehydmischung durch Leitung 3 oder verschiedene Aldehyde getrennt durch die Leitungen 3 und 4 zugemischt. Für die nachfolgend aufgeführten Beispiele wurde Pentanal als Edukt ausschließlich über Leitung 3 zugemischt. Die so erhaltene Mehrphasenmischuns 5 wird durch den Rohrreaktor 6 mit einer Länge von 3 m und einem Durchmesser von 17,3 mm gepumpt, der mit statischen Mischelementen mit einem hydraulischen Durchmesser von 2 mm versehen war. Die resultierende Mischung 7, bestehend aus dem Reaktionsprodukt, nicht umgesetztem Edukt und der Katalysatorphase werden im Flashbehälter 8 entspannt. Dabei fällt ein Kopfprodukt 9 und ein Sumpfprodukt 10 an. Der Flüssigkeitsstrom 10 wird in einen Phasentrennbehälter 11 geleitet. Hier wird die wässrige Katalysatorphase 2 abgetrennt und erneut dem Kreislauf zugeführt. Die über ein Wehr gelaufene organische Phase, die das Reaktionsprodukt enthält, wird aus Leitung 12 entnommen. Der Reaktor 6 wird bei ca. 2 bar unter Stickstoff betrieben, der Druck des Flashbehälters 8 ist in Tabelle 1 zu entnehmen.

Zur Gewährleistung einer konstanten Katalysatorzusammensetzung wird ein kleiner Teilstrom des Katalysators über Leitung 16 ausgeschleust und durch Zuführung von neuem Katalysator über Leitung 17 ausgeglichen.

Die außerhalb des Reaktors liegenden Wärmetauscher 13, 14 und 15 sind optional wobei die Reaktion selbst bzw. der Reaktor 6 adiabatisch betrieben werden. Der Wärmetauscher 13 kann zur Vorwärmung der Katalysatorphase dienen, insbesondere beim Anfahren des Reaktors. Mit dem Wärmetauscher 14 kann ein Teil der Reaktionswärme abgeführt werden, z. B. wenn das Reaktionsgemisch eine zu hohe Temperatur für die Kurzdestillation aufweist. Der Wärmetauscher 15 dient zur Kontrolle der Phasentrennung des Sumpfproduktes, da diese temperaturabhängig ist.

Die folgenden Beispiele beschreiben den Einsatz der oben beschriebenen kontinuierlichen Apparatur für das erfindungsgemäße Verfahren am Beispiel der Aldolkondensation von Pentanal zu 2-Propylheptenal (2PHal). Der Reaktor wurde mit einer Katalysatorbelastung von 400 kg/h bei Eigendruck der Reaktionsteilnehmer durchströmt. Die Temperatur des Katalysators, der Druck im Flashbehälter sowie der Eduktstrom (3) sind in Tabelle 1 angegeben. Die Tabelle 2 beschreibt das Kopf- und Sumpfprodukt des Flashbehälters.

Als Katalysatorlösung wurde Natronlauge mit einer Konzentration von 3 Gew.-% NaOH verwendet.

**Tabelle 1**

| **Beispiel** | **Pentanal (3) [g/h]** | **p (flash) [bar]** | **T [°C]** |
|---|---|---|---|
| 1 | 1150 | 0,80 | 110 |
| 2 | 6200 | 0,70 | 110 |
| 3 | 13000 | 0,50 | 110 |
| 4 | 1100 | 0,55 | 105 |
| 5 | 4900 | 0,45 | 105 |
| 6 | 7900 | 0,36 | 105 |
| 7 | 1050 | 0,20 | 98 |
| 8 | 4900 | 0,15 | 98 |

**Tabelle 2**

| | **Kopfprodukt (9)** | | | **Sumpfprodukt (10)** | |
|---|---|---|---|---|---|
| **Beispiel** | **Wasser [g/h]** | **Pentanal [g/h]** | **2PHal [g/h]** | **2Phal [g/h]** | **Pentanal [g/h]** |
| 1 | 109 | 88 | 26 | 940 | 0,9 |
| 2 | 594 | 433 | 101 | 5108 | 4,3 |
| 3 | 1293 | 614 | 276 | 11116 | 6,1 |
| 4 | 97 | 150 | 21 | 838 | 1,5 |
| 5 | 451 | 498 | 98 | 3878 | 5,0 |
| 6 | 777 | 409 | 167 | 6679 | 4,1 |
| 7 | 101 | 65 | 22 | 865 | 0,6 |
| 8 | 461 | 455 | 84 | 3965 | 4,6 |

Die Beispiele zeigen, daß die Reaktionswärme ausreicht, um das Reaktionswasser durch Flashung abzutrennen. Durch die Flashung sinkt der Gehalt an Pentanal im Produkt unter 0,2 Massen-%, so daß es ohne weitere Auftrennung weiterverarbeitet werden kann.

## Patentansprüche

1. Verfahren zur Herstellung von α, β-ungesättigten Ketoverbindungen durch basisch katalysierte Aldolkondensation von Aldehyden und/oder Ketonen mit 1 bis 15 Kohlenstoffatomen mit einer wäßrigen Katalysatorlösung in einer adiabatischen Reaktion, Trennen der so erhaltenen Reaktionsmischung in einer Kurzdestillation in ein Kopfprodukt, umfassend Wasser, Aldehyd und/oder Keton und in ein Sumpfprodukt, umfassend α, β-ungesättigte Ketoverbindungen und wäßrige Katalysatorphase,
**dadurch gekennzeichnet,**
**daß** die Verweilzeit der Reaktionsmischung in der Kurzdestillationsstufe maximal eine Minute beträgt.

2. Verfahren nach einem der Anspruch 1,
**dadurch gekennzeichnet,**
**daß** das Kopfprodukt in eine organische und wäßrige Phase getrennt wird.

3. Verfahren nach Anspruch 2,
**dadurch gekennzeichnet,**
**daß** die organische Phase des Kopfproduktes in die Aldolkondensationsreaktion zurückgeführt wird.

4. Verfahren nach einem der Ansprüche 1 bis 3,
**dadurch gekennzeichnet,**
**daß** das Sumpfprodukt in eine organische und die wäßrige Katalysatorphase getrennt wird.

5. Verfahren nach Anspruch 4,
**dadurch gekennzeichnet,**
**daß** die wäßrige Katalysatorphase in die Aldolkondensationsreaktion zurückgeführt wird.

6. Verfahren nach einem der Ansprüche 1 bis 5,
**dadurch gekenneichnet,**
**daß** die Alkolkondensation bei einem Druck von 1.1 bis 20 bar durchgeführt wird.

7. Verfahren nach einem der Ansprüche 1 bis 6,
**dadurch gekennzeichnet,**
**daß** die Kurzdestillation eine Flashdestillation, eine Destillation in einen Fallfilmverdampfer, eine Destillation in einem Dünnschichtverdampfer oder eine Destillation in einem kombinierten Fallfilm/Dünnschichtverdampfer ist.

8. Verfahren nach einem der Ansprüche 1 bis 7,
**dadurch gekennzeichnet,**
**daß** die Aldolkondensation in mindestens einem Rohrreaktor durchgeführt wird.

9. Verfahren nach einem der Ansprüche 1 bis 7,
**dadurch gekennzeichnet,**
**daß** die Aldolkondensation in mindestens einem Rührkessel durchgeführt wird.

10. Verfahren nach einem der Ansprüche 1 bis 9,
**dadurch gekennzeichnet,**
**daß** als wäßrige Katalysatorlösung Natronlauge eingesetzt wird.

## Claims

1. A process for preparing α,β-unsaturated keto compounds by base-catalysed aldol condensation of aldehydes and/or ketones having from 1 to 15 carbon atoms with an aqueous catalyst solution in an adiabatic reaction, separation of the reaction mixture obtained in this way in a rapid distillation into a top product comprising water, aldehyde and/or ketone and a bottom product comprising α,β-unsaturated keto compounds and aqueous catalyst phase, **characterized in that** the residence time of the reaction mixture in the rapid distillation stage is not more than one minute.

2. A process according to claim 1, **characterized in that** the top product is separated into an organic phase and an aqueous phase.

3. A process according to claim 2, **characterized in that** the organic phase of the top product is returned to the aldol condensation reaction.

4. A process according to any one of claims 1 to 3, **characterized in that** the bottom product is separated into an organic phase and the aqueous catalyst phase.

5. A process according to claim 4, **characterized in that** the aqueous catalyst phase is returned to the aldol condensation reaction.

6. A process according to any of claims 1 to 5, **characterized in that** the aldol condensation is carried out at a pressure of from 1.1 to 20 bar.

7. A process according to any one of claims 1 to 6, **characterized in that** the rapid distillation is a flash distillation, a distillation in a falling film evaporator, a distillation in a thin film evaporator or a distillation in a combined falling film/thin film evaporator.

8. A process according to any one of claims 1 to 7, **characterized in that** the aldol condensation is carried out in at least one tube reactor.

9. A process according to any one of claims 1 to 7, **characterized in that** the aldol condensation is carried out in at least one stirred vessel.

10. A process according to any one of claims 1 to 9, **characterized in that** sodium hydroxide solution is used as aqueous catalyst solution.

## Revendications

1. Procédé de préparation de composés cétoniques α, β-insaturés par condensation aldolique catalysée par une base, d'aldéhydes et/ou des cétones ayant de 1 à 15 atomes de carbone avec une solution aqueuse de catalyseur dans une réaction adiabatique, par séparation du mélange réactionnel ainsi obtenu dans une distillation de courte durée, en un produit de tête comprenant de l'eau, l'aldéhyde et/ou la cétone, et en un produit de pot comprenant les composés cétoniques α, β-insaturés et la phase aqueuse de catalyseur,
**caractérisé en ce que**
le temps de séjour du mélange réactionnel dans l'étape de distillation de courte durée s'élève au maximum à une minute.

2. Procédé selon la revendication 1,
**caractérisé en ce que**
le produit de tête est séparé dans une phase organique et aqueuse.

3. Procédé selon la revendication 2,
**caractérisé en ce que**
la phase organique du produit de tête est ramenée à la réaction de condensation aldolique.

4. Procédé selon l'une des revendications 1 à 3,
**caractérisé en ce que**
le produit de pot est séparé en une phase organique de catalyseur et la phase aqueuse de catalyseur.

5. Procédé selon la revendication 4,
**caractérisé en ce que**
la phase aqueuse de catalyseur est ramenée dans la réaction de condensation aldolique.

6. Procédé selon l'une des revendications 1 à 5,
**caractérisé en ce que**
la condensation aldolique est effectuée à une pression de 1,1 à 20 bars.

7. Procédé selon l'une des revendications 1 à 6,
**caractérisé en ce que**
la distillation atomique est une distillation flash, une distillation dans un évaporateur à film tombant, une distillation dans un évaporateur à couche mince, ou une distillation dans un évaporateur combiné film tombant/couche mince.

8. Procédé selon l'une des revendications 1 à 7,
**caractérisé en ce que**
la condensation aldolique est effectuée dans au moins un réacteur tubulaire.

9. Procédé selon l'une des revendications 1 à 7,
**caractérisé en ce que**
la condensation aldolique est effectuée dans au moins une chaudière d'agitation.

10. Procédé selon l'une des revendications 1 à 9,
**caractérisé en ce que**
comme solution aqueuse de catalyseur on met en oeuvre de la lessive de soude.
